# EUROPEAN PATENT APPLICATION

(11) **EP 1 714 655 A1**
(43) Date of publication of application: **25.10.2006**
(21) Application number: 05384002.1
(22) Date of filing: 22.04.2005
(51) Int. Cl.: A61K 31/529, A61K 31/52, A61K 31/465, A61P 25/34

(54) **Use of sodium channel blockers and their analogues for the treatment of nicotine dependency**

(71) Applicant: LABORATORIOS DEL DR. ESTEVE, S.A., 08041 Barcelona (ES)
(72) Inventor: Buschmann, Helmut, Dr., Esplugas de Llobregat (Barcelona) (ES); Farre Gomis, Antonio, 08037 Barcelona (ES); Vela Hernandez, Jose Miguel, 08028 Barcelona (ES)

(57) **Abstract**

The present invention refers to the use of a sodium channel blocker such as tetrodotoxin or saxitoxin, their analogues and derivatives, their physiologically acceptable salts, as well as their possible combination with nicotine in medicinal products for human and/or animal therapeutics for the treatment of nicotine dependency.

## Description

### Field of the invention

The present invention refers to the use of a sodium channel blocker such as tetrodotoxin or saxitoxin, their analogues and derivatives as well as their physiologically acceptable salts, in medicinal products for human therapeutics and/or animal research for the treatment of nicotine dependency.

### Background of the invention

Nicotine is a natural product that can be isolated from the leaves of the tobacco plant. Tobacco has been consumed for centuries in Mezo- and South America, valued for its effects after smoking. It is now consumed worldwide. Even though its effect on the body are not as devastating as that of other drugs which produce dependency it nevertheless has developed into an enormous health care problem with lung cancer being one of the main causes for death.

For the moment any de-addiction treatment from nicotine mainly involves behavioral therapy sometimes helped by medication. Accordingly there is a high need for an effective and successful de-addiction therapy especially for useful agents in the pharmacotherapy of addiction from nicotine.

Therefore the subject of this invention is the use of a sodium channel blocker, and/or one of its derivatives for the production of a drug for treatment of addiction to nicotine, to treat dependency upon nicotine or ameliorate the effects of a de-addiction treatment from nicotine. The sodium channel blocker is optionally used in the form of its racemate, pure stereoisomers, especially enantiomers or diastereomers or in the form of mixtures of stereoisomers, especially enantiomers or diastereomers, preferably in any suitable ratio; in neutral form, in the form of an acid or base or in form of a salt, especially a physiologically acceptable salt, or in form of a solvate, especially a hydrate.

So it is now established that a sodium channel blocker, such as tetrodotoxin (TTX) or saxitoxin (STX), could help in de-addiction treatment of nicotine.

The term "sodium channel blocker" mentioned in this application is defined as a compound that specifically binds to and specifically inhibits sodium channels, which are classified as either TTX-resistant or TTX-sensitive. The term TTX-resistant" and TTX-sensitive refers to a difference in the tightness of TTX binding, with the TTX resistant channel having a binding constant as mentioned in Hunter et al.,Current Opinion in CPNS Investigational Drugs 1 (1), 1999 as well as Clare et al. DDT, 5 (11), 2000, 506-520 included here by reference and the TTX sensitive channel having a binding constant as mentioned in Hunter et al., Current Opinion in CPNS Investigational Drugs 1 (1), 1999 as well as Clare et al. DDT, 5 (11), 2000, 506-520. A preferred sodium channel blocker thus binds to a sodium channel with a IC₅₀ of less than 200 µM, preferably less than 100 µM or with an IC₅₀ of 2 µM. Said inhibition refers to suppression or modification of any downstream effect caused by activation of said sodium channels. More preferably, the term "sodium channel blocker" mentioned in this invention refers to compounds binding to an alpha subunit of sodium channels, especially TTX-resistant or TTX-sensitive sodium channels. More preferably, the term "sodium channel blocker" mentioned in this invention refers to compounds binding to either a SS1 or SS2 region of an alpha subunit of sodium channels, especially TTX-resistant or TTX-sensitive sodium channels. Preferred sodium channel blockers for use in this invention are tetrodotoxin and saxitoxin which both specifically inhibit said sodium channels.

The term "analogues" as used in this application is defined here as meaning a chemical compound that is a derivative of a compound which has similar biochemical activity to that compound. "Analogues" of TTX and STX bind to the same site on the alpha subunit of sodium channels as does TTX and STX.

The term "derivatives" as used in this application is defined here as meaning a chemical compound having undergone a chemical derivation such as substitution or addition of a further chemical group to change (for pharmaceutical use) any of its physico-chemical properties, such as solubility or bioavailability. Derivatives include so-called prodrugs, e.g. ester and ether derivatives of an active compound that yield the active compound per se after administration to a subject.

Examples of well known methods of producing a prodrug of a given acting compound are known to those skilled in the art and can be found e.g. in Krogsgaard-Larsen et al., Textbook of Drugdesign and Discovery, Taylor & Francis (April 2002).

Tetrodotoxin (alternatively in the context of this application abbreviated TTX), also known as Ti Qu Duo Xin, is an alkaloid found in puffer fish (Tetradontiae). The chemical name is Octahydro-12-(Hydroxymethyl)-2-imino-5, 9, 7, 10a-dimethano-10aH-[1,3]dioxocino[6,5-d]pyrimidine-4,7,10,11,12-pentol with a molecular formula C₁₁H₁₇N₃O₈ and a Molecular weight of 319.27. It is a potent non-protein neurotoxin and an indispensable tool for the study of neurobiology and physiology. Tetrodotoxin (TTX) is a marine organic toxin which is mainly found in testicles, ovaries, eggs, livers, spleens, eyeballs, and blood of puffer fish as well as in diverse animal species, including goby fish, newt, frogs and the blue ringed octopus and even in marine alga. Several processes for producing TTX are known. Usually TTX is extracted from marine organisms (e.g. JP 270719 Goto and Takahashi) but besides numerous others methods of synthesis are also described (and used for the preparation of tetrodotoxin in connection to this invention) in US 6,552,191, US6,478,966, US 6,562,968 or 2002/0086997, all of which are included here by reference. Tetrodotoxin is a well known compound described for example in WO02/22129 as systemically acting as analgesic. For one of the many descriptions of TTX it is recommended turn to e.g. Tu, Anthony (Ed.) Handbook of Natural Toxins, Vol. 3: Marine Toxins and Venoms, 1988, 185-210 as well as Kao (1966), Pharmacol. Rev. 18:997 - 1049 and others.

The usefulness of TTX in treatment of addiction to alkaloids and synthetic non-amino acid nitrogen-containing compounds is already claimed in EP 0 750 909 and also described in CN 1145225 and CN 1227102. WO 03/099301 describes aerosol, spray or gasoloid formulation for addiction treatment in regards to alkaloids with the majority being opioids. Even so, this art does not touch the current invention because nicotine dependency from which millions of smoker's suffer never is summarized under drug addiction. Therefore, it came as a surprise when during our experiments it was discovered that TTX could help in treating nicotine addiction.

In the scientific literature however, the main use of TTX (and to a lesser extent) saxitoxin has been its use as pharmacological research tools in neurobiology, pharmacology and electrophysiology. Respective exemplary experiments referring to TTX were performed in the context of the actions of heroin (Rita et al, 2002), cocaine (Erb et al., 2001; McLaughlin et al., 2001), ethanol (Yan et al., 2003) morphine (De Rover et al., 2005) and nicotine (Benwell et al., 1993).
A recent study performed by Corrigal et al (Corrigal et al., 2002) TTX was used as a research tool in the context of nicotine dependence. In this study TTX was infused intracranially into the pedunculopontine tegmental nucleus, thereby provoking a temporary functional lesion of said area with the aim to block cholinergic neuron signalling. The amount of TTX used in this neurophysiological study is extremely high compared to a therapeutic dosage when one considers the weight of this respective lesioned brain area. This study shows only that TTX-mediated functional deletion of a special cell type such as cholinergic neurons in a cerebral nucleus leads to a modification of behavior of drug-dependent rats. Consequently, a person skilled in the art would consider the study performed by Corrigal et al. as being out of the scope of the present invention, both in the sense of mechanism as well as in the sense of TTX usage. In addition we consider especially that the prior art as a whole does not anticipate use of TTX, or any other sodium channel blocker, in treatment of subjects having an established addiction to nicotine.

The phrase "its (tetrodoxin's) derivatives" according to this invention is defined - using the definition of US 6,030,974 (included here by reference) - as meaning amino perhydroquinazoline compounds having the molecular formula C₁₁H₁₇N₃O₈. "Tetrodoxin's derivatives" according to this invention encompasses compounds described in US 5,846,975 (included here by reference) as amino hydrogenated quinazolines and derivatives including the substances set forth from column 3 line 40 to column 6 line 40. Specifically exemplified "derivatives of tetrodotoxin" according to this invention are including but are not limited to anhydro-tetrodotoxin, tetrodaminotoxin, methoxytetrodotoxin, ethoxytetrodotoxin, deoxytetrodotoxin and tetrodonic acid, 6 epi-tetrodotoxin, 11-deoxytetrodotoxin as well as the hemilactal type TTX derivatives (e.g. 4-*epi*-TTX, 6-*epi*-TTX, 11-*deoxy*-TTX, 4-*epi*-11-*deoxy-*TTX, TTX-8-*O*-hemisuccinate, chiriquitoxin, 11-*nor*-TTX-6(S)-ol, 11-*nor*-TTX-6(R)-ol, 11-*nor*-TTX-6,6-diol, 11-*oxo*-TTX and TTX-11-carboxylic acid), the lactone type TTX derivatives (e.g. 6-*epi*-TTX (lactone), 11-*deoxy*-TTX (lactone), 11-*nor*-TTX-6(S)-ol (lactone), 11-*nor*-TTX-6(R)-ol (lactone), 11-*nor*-TTX-6,6-diol (lactone), 5-*deoxy*-TTX, 5,11-*dideoxy*-TTX, 4-*epi*-5,11-*didroxy*-TTX, 1-*hydroxy*-5,11-*dideoxy-*TTX, 5,6,11-*trideoxy*-TTX and 4-*epi*-5,6,11-*trideoxy*-TTX) and the 4,9-anhydro type TTX analogs (e.g. 4,9-*anhydro*-TTX, 4,9-*anhydro*-6-*epi*-TTX, 4,9-*anhydro*-11-*deoxy*-TTX, 4,9-*anhydro*-TTX-8-*O*-hemisuccinate, 4,9-*anhydro*-TTX-11-*O-*hemisuccinate). The typical derivatives of TTX possess only 1/8 to 1/40 of the toxicity of TTX in mice, based upon bioassay in mice. It has been observed that these derivatives produce joint action, and do not interact adversely. Examples of TTX derivatives include novel TTX derivatives isolated from various organisms, as well as those that are partially or totally chemically synthesized (see e.g., Yotsu, M. et al. Agric. Biol. Chem., 53(3):893-895 (1989)).

According to U. S. Patent No. 6,030,974,"saxitoxin" or "STX" refers to a compound comprising a tetrahydropurine moiety composed of two guanidine units fused together in a stable azaketal linkage, having a molecular formula CloHI7N704 (mol. wt. 299.30) and to derivatives thereof, including but not limited to hydroxysaxitoxins and neosaxitoxin. Bower et al., Nonprotein Neurotoxins, Clin. Toxicol. 18 (7): 813-863 (1981).

In connection with this invention "neutral form" refers either to a non-ionic form or to a neutrally net charged form, for example a Zwitter-Ion at its isoelectric point.

The term "salt" according to this invention is to be understood as meaning any form of the active compound according to the invention in which this compound assumes an ionic form or is charged and - if applicable - is also coupled with a counter-ion (a cation or anion). By this are also to be understood complexes of the active compound with other molecules and ions, in particular complexes which are complexed via ionic interactions. As preferred examples of salts this includes the acetate, mono-trifluoracetate, acetate ester salt, citrate, formate, picrate, hydrobromide, monohydrobromide, monohydrochloride or hydrochloride.

The term "physiologically acceptable salt" in the context of this invention is understood as meaning a "salt" (as defined above) of at least one of the compounds according to the invention which are physiologically tolerated by humans and/or mammals.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate.

The term "effects of a de-addiction treatment" in the context of this invention is understood as including any side effect coming with any de-addiction treatment, especially any kind of withdrawal syndrome.

The term "ameliorate" in the context of this invention is understood as meaning any improvement on the situation of the patient treated - either subjectively (feeling of or on the patient) or objectively (measured parameters).

The term "treatment" or "to treat" in the context of this specification means administration of a compound or formulation according to the invention to prevent, ameliorate or eliminate one or more symptoms associated with de-addiction from nicotine. Such symptoms can arise from withdrawal, or can be associated with relapse behavior, such as a craving of a subject for nicotine. "Treatment" also encompasses preventing, ameliorating or eliminating the physiological sequelae of removal of nicotine from an addicted subject that may cause the exhibited symptoms perceived by the subject. "Treatment" also encompasses a reduction in the amount of nicotine that is consumed by a subject.

Furthermore, the terms "to treat" or "treatment" according to this invention include the treatment of symptoms of de-addiction, nicotine dependency or nicotine withdrawal, especially certain subtypes of de-addiction, drug dependency or nicotine withdrawal, the treatment of the consequences causing the symptoms, the prevention or the prophylaxis of the symptoms of de-addiction, or nicotine withdrawal, especially certain subtypes of de-addiction, or nicotine withdrawal, the prevention or prophylaxis causing the symptoms of de-addiction, or nicotine withdrawal, as well as the prevention or the prophylaxis of the consequences causing the symptoms.

The wording "nicotine replacement therapy" used in this application refers to any form of nicotine replacement that can help lessen the urge to smoke. "Nicotine replacement therapy" also includes the utilization of nicotine patches, gums, nasal sprays and any other form of nicotine application, either oral or parenteral.

For treatment of human subjects, the dose administered is normally between 5 and 4000 µg/day when tetrodotoxin, or a derivative or analog thereof is given. When TTX is administered the dosage is usually from 5 to 4000 µg/day. Administration is typically twice per day and on such a schedule each administered dose will typically contain from 2.5 to 2000 µg of TTX or of a derivative or analog thereof. Preferably, from 2.5 to 500 ug is administered per dose, more preferably from 50 to 500 µg per dose, or from 50 to 400 µg per dose. In some administrations, from 2.5 to 30 µg per dose is given. The dosage will vary with the route of administration, with intravenous administration typically utilizing a lower dosage than oral administration.

A substance named as an "active ingredient" will have a purity of at least 97%. For example, a formulation said to have "500 µg of TTX as the active ingredient" may contain as much as 15 µg of anhydrotetrodotoxin as an impurity. On the other hand, a formulation said to have "500 µg of TTX and 500 µg of anhydrotetrodotoxin as active ingredients" will contain at least 485 ug of TTX and 485 µg of anhydrotetrodotoxin, but may contain as much as 30 µg of other substances as impurities of the active ingredients. Of course, substances named as other components of a formulation are not included when the purity of the active ingredient is considered.

In a preferred embodiment of the invention the use according to the invention is restricted to treat dependency upon nicotine or ameliorate the effects of a treatment for de-addiction from nicotine.

In a preferred embodiment of the invention the use according to the invention is restricted to tetrodotoxin as the active ingredient. The TTX is optionally provided in the form of its racemate, pure stereoisomers, especially enantiomers or diastereomers or in the form of mixtures of stereoisomers, especially enantiomers or diastereomers, preferably in a suitable ratio; in neutral form, in the form of an acid or base or in form of a salt, especially a physiologically acceptable salt, or in form of a solvate, especially a hydrate.

In a preferred embodiment of the invention the use according to the invention is restricted to tetrodotoxin as the active ingredient, preferably in any suitable ratio in neutral form or as a salt, especially a physiologically acceptable salt.

In a preferred embodiment of the invention tetrodotoxin, any of its derivatives and/or one of its analogues is used as the active ingredient in an amount between 10 µg/day and 4 mg/day.

In a preferred embodiment of the invention tetrodotoxin, its derivative or its analogue is isolated from a biological source, preferably from fish, especially puffer fish.

In a preferred embodiment of the invention tetrodotoxin, its derivative or its analogue is synthetic.

In a preferred embodiment of the invention, nicotine in any type of formulation as mentioned in this application, is administered in combination with tetrodotoxin or saxitoxin, their derivatives or analogues. The amount of nicotine is typically chosen according to the needs of nicotine of the patient (as well-known in the art for de-addiction using only nicotine) and preferably decreases over time. It is desirable to ultimately administer no nicotine.

In a preferred embodiment of the invention, nicotine is administered in combination with tetrodotoxin or saxitoxin, their derivatives or analogues, either in form of a patch, a gum, a nasal spray, an inhaler or any other form of topical administration.

The term "in combination" includes but does not exclusively mean the concomitant administration of compounds, but also the sequential administration of each of the compounds, as well as administration of one compound, followed by administration of the other compound, whereas the time interval between the two administrations can be between 1 min and 1 week. Additionally, "in combination" refers to a formulation containing all compounds in its respective carrier such as a tablet, capsule, a pill or a gum. Most preferably, "in combination" means a fixed combination of compounds in a single dosage form such as a tablet, a pill , a capsule or a gum.

In general a preferred embodiment of the invention is a combination comprising a substance A selected from the group of sodium channel blockers, and/or one of its derivatives, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate
and
substance B, selected from nicotine as well as its derivatives.

A preferred embodiment of this invention is a combination wherein substance A selected from the group of sodium channel blockers is tetrodotoxin, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate.
Another preferred embodiment of the invention is a medicament comprising a substance A selected from the group of sodium channel blockers, and/or one of its derivatives, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate
and
substance B, selected from nicotine as well as its derivatives.

Another preferred embodiment of the invention is the use of an above-mentioned combination for the production of a drug for the treatment of nicotine dependence, de-addiction of nicotine dependence or amelioration of the effects of a de-addiction treatment from nicotine.

A preferred embodiment of this invention is a use according to this invention wherein the drug is manufactured to be administered parenterally and/or orally.

A preferred embodiment of this invention is a use according to this invention wherein the drug is manufactured to be administered in form of a patch, a gum, a nasal spray, an inhaler or any other form of topical administration.

Another preferred embodiment of the invention is a dosage form of TTX, and/or of an analog and/or derivative thereof, for the treatment of nicotine addiction.

Anothere preferred embodiment of the invention is a the use of TTX, and/or of an analog and/or derivative thereof, to prepare a medicament for treatment of nicotine addiction.

Another preferred embodiment of the invention is a kit comprising a drug comprising a substance A selected from the group of sodium channel blockers, and/or one of its derivatives, optionally in the form of its racemate, pure stereoisomers, especially enantiomers or diastereomers or in the form of mixtures of stereoisomers, especially enantiomers or diastereomers, in any suitable ratio; in neutral form, in the form of an acid or base or in form of a salt, especially a physiologically acceptable salt, or in form of a solvate, especially a hydrate
and a drug comprising substance B, selected from nicotine as well as its derivatives.

Any formulation or pharmaceutical composition according to the invention contains the active ingredient (nicotine, saxitoxin or TTX, its derivatives and/or its analogues) as well as optionally at least one auxiliary material and/or additive.

The auxiliary material and/or additive can be one ore more of conserving agents, emulsifiers and/or carriers for parenteral application. The selection of these auxiliary materials and/or additives and of the amounts to be used depends upon how the pharmaceutical composition is to be administered. Examples include especially parenteral routes like intravenous subcutaneous or intramuscular administration.

Routes of administration of tetrodotoxin its derivatives and its analogues can include intramuscular injection, intraveneous injection, subcutaneous injection, sublingual, bucal, patch through skin, oral ingestion, implantable osmotic pump, collagen implants, aerosols or suppository.

Included in this invention are especially also methods of treatments of a patient or a mammal, including man, suffering from addiction from nicotine, the method comprising the administration of tetrodotoxin, of its derivatives and/or of its analogues optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate. It is also preferred if the method of treatment is restricted to tetrodotoxin, optionally in the form of its racemate, pure stereoisomers, especially enantiomers or diastereomers or in the form of mixtures of stereoisomers, especially enantiomers or diastereomers, preferably in any suitable ratio; in neutral form, in the form of an acid or base or in form of a salt, especially a physiologically acceptable salt, or in form of a solvate, especially a hydrate. It is also preferred if the method of treatment is restricted to tetrodotoxin, in neutral form or as a salt, especially a physiologically acceptable salt, whereas preferably tetrodotoxin, its derivative and/or one of its analogues is used in an amount between 10 µg/day and 4 mg/day, is isolated from a biological source, preferably from fish, especially puffer fish, or is synthesized.

The present invention also includes articles of manufacture that include a dosage form of TTX, or of one or more of its derivatives and/or analogs, or a mixture of TTX and one or more of its derivatives and /or analogs, packaged together with written material that urges the administration of the dosage form for the treatment of nicotine addiction. The dosage form provided with the article of manufacture according to the invention is preferably one that is appropriate for oral administration or administration by a "patch" that comprises substances for carrying the active ingredients through the skin. The dosage form included in the' article of manufacture may be one appropriate for parenteral injection.
Articles of the scientific periodical and patent literature cited herein are hereby incorporated by reference in their entirety by such citation.

The examples and figures in the following section describing pharmacological trials are merely illustrative and the invention cannot be considered in any way as being restricted to these applications.

### Examples:

### Example 1:

### Example formulation of an injectable (im/iv) solution of TTX

| | |
|---|---|
| Tetrodotoxin (TTX) (powdered material) | 15 mg |
| 0.5% diluted acetic acid | 1 ml |
| Acetic Acid - actetate buffer solution (pH=3-5) | 50 ml |
| Water for injection c.s.p., add to | 1000 ml |

The TTX is dissolved in the 0.5% acetic acid and then the TTX solution is further diluted in the acetate buffer and finally diluted with the water for injection. The formulation is sterilized by ultrafiltration and packaged in injection bottles.

The dosage of TTX for injection is 30 µg in 2 ml.

### Example 2:

### Nicotine dependence and withdrawal

Nicotine dependence was induced by using Alzet osmotic minipumps (Model 2001) (Alzet® , Cupertino, CA, USA). These minipumps, implanted subcutaneously under brief ether anaesthesia, contained saline or nicotine solutions and delivered a constant subcutaneous flow in a rate of 1 µl/h. The concentration of nicotine was adjusted to compensate for differences in the body weight of the mice. Thus, the average-weighed mice received a dose of approximately 25 mg/kg/day nicotine during six days. Nicotine withdrawal syndrome was precipitated six days after minipump implantation by injection of the nicotinic receptor antagonist, mecamylamine (1 mg/kg, sc). Tetrodotoxin (3 µg/kg, sc) or vehicle was administered 30 min before mecamylamine injection. The somatic signs of withdrawal were evaluated immediately after mecamylamine injection during a period of 30 min. The number of wet dog shakes, front paw tremors, writhes and scratches was counted. Body tremor, ptosis, teeth chattering, genital licks and piloerection were scored 1 for appearance or 0 for non-appearance within each 5 min time. The locomotor activity over 5 min periods was rated 0, 1 or 2 (0 for inactivity, 1 for low activity and 2 for normal activity). A global withdrawal score was calculated for each animal by giving each individual sign a relative weight (Castañé et al., Neuropharmacology 43: 857, 2002).

The effects induced by the administration of tetrodotoxin on the expression of nicotine physical dependence were evaluated. Animals were distributed as follows:

| | |
|---|---|
| Group 1 (n = 10): | chronic vehicle + acute vehicle |
| Group 2 (n = 10): | chronic nicotine + acute vehicle |
| Group 3 (n = 10): | chronic vehicle + acute tetrodotoxin |
| Group 4 (n = 10): | chronic nicotine + acute tetrodotoxin |

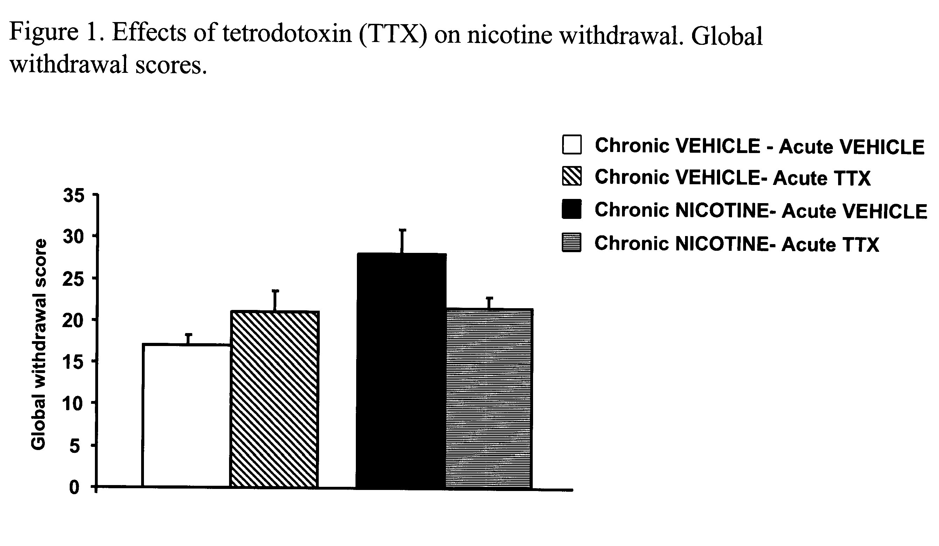

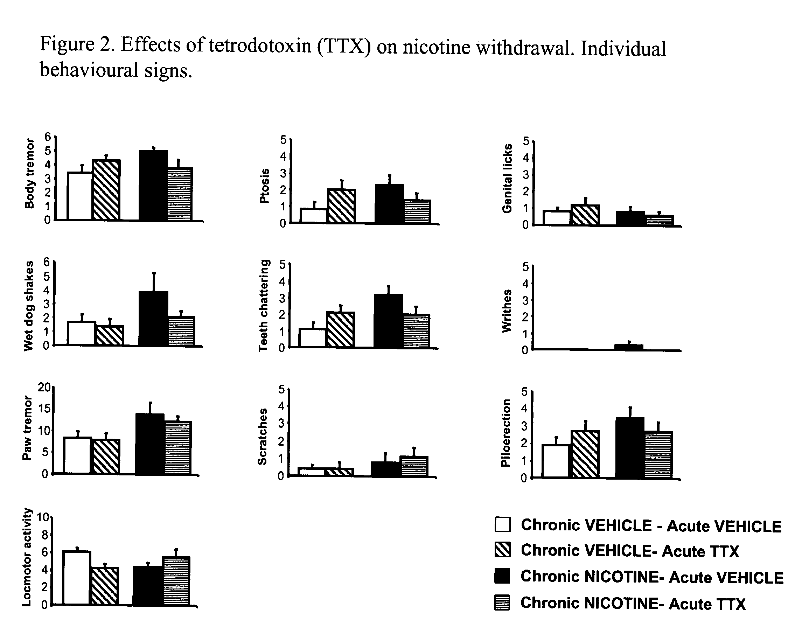

The results shown in figure 1 and 2 clearly demonstrate that administration of tetrodotoxin attenuates the somatic expression of nicotine withdrawal syndrome significantly.

## Claims

1. Use of a sodium channel blocker, and/or of one of its derivatives, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate, for the production of a drug for addiction treatment from nicotine, to treat dependency from nicotine or ameliorate the effects of a de-addiction treatment from nicotine.

2. Use according to claim 1 **characterized in that** the use is restricted to tetrodotoxin or to at least one of its derivatives and/or analogues or a mixture thereof, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate.

3. Use according to any of claims 1 or 2 **characterized in that** the use is restricted to tetrodotoxin in neutral form or as a salt, especially a physiologically acceptable salt.

4. Use according to any of claims 2 or 3, **characterized in that** tetrodotoxin, at least one of its derivatives and/or one of its analogues is used in an amount between 10 µg/day and 4 mg/day.

5. Use according to any of claims 2 to 4, **characterized in that** tetrodotoxin, its derivative or its analogue is isolated from a biological source, preferably from fish, especially puffer fish.

6. Use according to any of claims 2 to 4, **characterized in that** tetrodotoxin, its derivative or its analogue is synthetic.

7. A combination comprising a substance A selected from the group of sodium channel blockers, and/or one of its derivatives, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate
and
substance B, selected from nicotine as well as its derivatives.

8. A combination according to claim 7, **characterized in that** substance A selected from the group of sodium channel blockers is tetrodotoxin, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate.

9. A medicament comprising a substance A selected from the group of sodium channel blockers, and/or one of its derivatives, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate
and
substance B, selected from nicotine as well as its derivatives.

10. Use of a combination according to claim 7 for the production of a drug for the treatment of nicotine dependence, de-addiction of nicotine dependence or amelioration of the effects of a de-addiction treatment from nicotine.

11. Use according to claim 10, **characterized in that** the drug is manufactured to be administered parenterally and/or orally.

12. Use according to claims 9 or 10, **characterized in that** the drug is manufactured to be administered in form of a patch, a gum, a nasal spray, an inhaler or any other form of topical administration.

13. A dosage form of TTX, and/or of an analog and/or derivative thereof, for the treatment of nicotine addiction.

14. Use of TTX, and/or of an analog and/or derivative thereof, to prepare a medicament for treatment of nicotine addiction.

15. A kit comprising a drug comprising a substance A selected from the group of sodium channel blockers, and/or one of its derivatives, optionally in the form of its racemate, pure stereoisomers, especially enantiomers or diastereomers or in the form of mixtures of stereoisomers, especially enantiomers or diastereomers; preferably in any suitable ratio; in neutral form, in the form of an acid or base or in form of a salt, especially a physiologically acceptable salt, or in form of a solvate, especially a hydrate
and a drug comprising substance B, selected from nicotine as well as its derivatives.
